# EUROPÄISCHE PATENTSCHRIFT

(11) **EP 0 730 463 B1**
(45) Veröffentlichungstag und Bekanntmachung des Hinweises auf die Patenterteilung: **02.02.2000**
(21) Anmeldenummer: 95901442.4
(22) Anmeldetag: 26.11.1994
(51) Int. Cl.: A61K 33/14, A61K 33/00

(54) **VERWENDUNG VON LITHIUMVERBINDUNGEN ZUR BEHANDLUNG UND PROPHYLAXE DES MORBUS ALZHEIMER**
USE OF LITHIUM COMPOUNDS IN THE TREATMENT AND PREVENTION OF ALZHEIMER'S DISEASE
UTILISATION DE COMPOSES LITHIUM DANS LE TRAITEMENT ET LA PROPHYLAXIE DE LA MALADIE D'ALZHEIMER

(30) Priorität: 26.11.1993 DE 4340272; 26.11.1993 DE 4340273
(43) Veröffentlichungstag der Anmeldung: 11.09.1996
(73) Patentinhaber: Lehmann, Karla, 70184 Stuttgart (DE)
(72) Erfinder: Lehmann, Karla, 70184 Stuttgart (DE)
(74) Vertreter: Weller, Wolfgang, Dr.rer.nat.
(86) Internationale Anmeldenummer: EP9403921
(87) Internationale Veröffentlichungsnummer: WO9514481

(56) Entgegenhaltungen:
- EP-A- 0 234 733
- EP-A- 0 289 204
- EP-A- 0 500 006
- WO-A-92/00737
- J. CLIN. PSYCHOPHARMACOL., Bd.2, Nr.1, 1982 Seiten 71 - 72 W.W. HAVENS ET AL. 'Successful treatment of dementia with lithium'
- PSYCHOPHARMACOLOGY (BERL) (GERMANY, WEST), MAY 9 1977, VOL. 52, NO. 3, PAGE(S) 307-9, SAMPLES JR ET AL 'Lethal effects of physostigmine plus lithium in rats.'

## Beschreibung

Die Erfindung betrifft die Verwendung von zumindest einer Lithiumverbindung zur Herstellung eines Arzneimittels zur Prophylaxe und Behandlung von Morbus Alzheimer.

Die Alzheimersche Erkrankung ist eine etwa im 5.-6. Lebensjahrzehnt beginnende, präsenile, fortschreitende neurodegenerative Erkrankung. Mit zunehmendem Lebensalter steigt die Häufigkeit dieser Erkrankung an. So sind etwa 0,5 % der Population im Alter von 65 Jahren, 10 % der Population im Alter von 85 Jahren von dieser Erkrankung befallen (siehe Breteler, M.M.B. et al., Epidemiol. Rev. 14: 59-82, 1992). Bei einer weiteren Erhöhung des durchschnittlichen Lebensalters der Bevölkerung ist damit zu rechnen, daß auch die Anzahl der Alzheimer-Patienten wächst. Bis heute existiert weder eine effektive symptomatische Behandlung, noch gibt es Möglichkeiten, das Fortschreiten dieser Erkrankung aufzuhalten. Therapeutisch wirksame, weit mehr jedoch prophylaktisch einsetzbare Arzneimittel gewinnen gerade für die Behandlung dieser chronischen Erkrankung besondere Bedeutung.

Forschungen der letzten Jahre ergaben, daß es bei dieser Erkrankung zu einer Atrophie des Kortex und zu einem Zellverlust des Hippokampus, zu einer Reduktion cholinerger Zellen im basalen Vorderhirn sowie ihrer cholinergen Projektionen kommt, woraus ein globales cholinerges Defizit resultiert. Außerdem wurden extraneuronale Plaques, bestehend aus dem unlöslichen Amyloid-β-Protein (ABP) nachgewiesen. Vorstufe dieses β-Amyloids ist das Amyloid-Präkursor-Protein (APP), dessen basale Freisetzung nach Aktivierung postsynaptischer M₁/M₃-Rezeptoren über eine Aktivierung der Proteinkinase C erfolgt (Lambrecht, G. et al., Medicinal chemistry approaches in Alzheimer's disease: Focus on cholinergic mechanisms, Vortrag, Intern. Cong. Pharmac., Japan, 1993).

Aus J.Clin.Psychopharmacol. Band 2, Nr. 1, 1982, Seiten 71 bis 72, W.W. Havens et al. "Successful treatment of dementia with lithium", ist die Behandlung von Manie durch Lithiumcarbonat bekannt.

Aus dem Dokument EP,A,0 234 733 ist die Behandlung von Morbus Alzheimer mit Lithiumverbindungen bekannt.

In dem Dokument EP,A,0 289 204 sind Lithiumsalze von mehrfach ungesättigten Fettsäuren zur Behandlung von Morbus Alzheimer beschrieben.

Aus dem Dokument WO,A,92 00737 ist bekannt, eine Mischung aus Physostigmin und Aminopyridin zur Behandlung des Morbus Alzheimer einzusetzen.

Dokument EP,A,0 500 006 beschreibt die Verwendung des Acetylcholinesterasehemmers Tacrin zur Behandlung von Morbus Alzheimer.

Es sind schon Therapieversuche bekannt (Summers, W.K. et. al., Oral tetrahydroaminoacridine in long term treatment of senile dementia, Alzheimer type, New Engl. Journal Medicine 315: 1241-1245, 1986), die Azetylcholin-Präkursoren, Stimulatoren der Azetylcholinfreisetzung und Azetylcholinesterasehemmer einschließen, wovon letztere am ehesten erfolgversprechend erschienen. Von Tacrin, einem reversiblen, nicht-kompetetiven Azetylcholinesteraseinhibitor, wurde eine erhöhte Wirksamkeit im Vergleich zu Physostigmin bei Patienten bei Morbus Alzheimer beschrieben.

Allerdings wurde eine nicht zufriedenstellende Wirksamkeit von Azetylcholinesterasehemmern in Monotherapie festgestellt.

Aufgabe der vorliegenden Erfindung ist, neue Möglichkeiten zu schaffen, um die Entstehung und das weitere Fortschreiten der Alzheimerischen Erkrankung zu bekämpfen.

Erfindungsgemäß wird die Aufgabe durch die Verwendung einer fixen Kombination aus zumindest einer Lithiumverbindung und zumindest einem Acetylcholinesterasehemmer zur Herstellung eines Arzneimittels zur Prophylaxe und Behandlung des Morbus Alzheimer gelöst.

Die Wirkungen von Lithium beruhen insbesondere auf
1. der Aktivierung der zentralen cholinergen Transmission,
2. der Senkung des intrazellulären IP₃-Pools (rationale Antiamyloidtherapie)
3. und einer bevorzugten Verteilung von Lithium in Regionen, die beim Morbus Alzheimer ein cholinerges Defizit aufweisen.

In Dosen im Bereich derer, die heute bei anderen Indikationen Verwendung finden, sind unerwünschte Reaktionen von Lithiumverbindungen selten und begrenzbar. Die umfangreichen Erfahrungen, die im Umgang mit Lithiumverbindungen bei anderen Indikationen über Jahrzehnte hinweg gewonnen wurden, sind für die sichere Anwendung beim Morbus Alzheimer nutzbar. Insgesamt erweist sich Lithium als ideales und physiologisches Arzneimittel zur Prophylaxe des Morbus Alzheimer und zur Verhütung des Fortschreitens dieser Erkrankung.

Als vorteilhaft haben sich Lithiumverbindungen ausgewählt aus der Gruppe bestehend aus Lithiumazetat, -adipinat, -aspartat, -hydrogenaspartat, -benzoat, -bromid, -chlorid, -glukonat, -karbonat, -orotat, -salizylat, -sulfat, -zitrat erwiesen.

Untersuchungen aus den späten siebziger Jahren deuten darauf hin, daß Lithium zu einer Aktivierung des cholinergen Transmissionsystems führt (Greil, W. et. al., Lithium: Grundlagen und Therapie, in Langer, G., Heimann, H. Psychopharmaka: 161-202, 1983 Springer-Verlag, Wien-New York).

Es wurden ferner physostigminsynergistische Effekte von Lithium bis hin zur erhöhten Toxizität im Tierexperiment nachgewiesen, die durch Atropin antagonisierbar sind (Van Calker, D. et al.: Biochemische und zellphysiologische Effekte von Lithiumionen in "Die Lithiumtherapie", Hrsg.: Müller-Oerlinghausen, B. und W. Greil, Springer-Verlag, Berlin-Heidelberg-New York-Tokyo, 5-34, 1986).

Hinzu kommt, daß nach systemischer Mehrfachapplikation von Lithiumverbindungen an Versuchstieren höhere Lithiumkonzentrationen im Kortex, Hippokampus und Striatum als in anderen Hirnregionen auftraten (Lehmann, K.: Die Kinetik von Lithium im Serum, in der Leber und im Gehirn der Ratte, Acta Biol. Med. Germ. 34: 1043-1047, 1975).

Dies sind insbesondere die Regionen, in denen bei Morbus Alzheimer-Patienten ein cholinerges Defizit nachgewiesen werden konnte.

Zweckmäßigerweise übersteigt die Dosierung der Lithiumverbindungen die bei anderen Indikationen angewandten üblichen Tagesdosen nicht. Das entsprechende Arzneimittel wird systemisch, d.h. insbesondere oral, intravenös oder anders verabreicht.

Die Lithiumverbindungen lassen sich in üblicher pharmazeutischer Weise zu den gewünschten Darreichungsformen konfektionieren, wobei Trägerstoffe, Verdünnungsmittel und/oder Hilfsstoffe eingesetzt werden können.

Durch die nunmehr vorgeschlagene fixe Kombination einer Lithiumverbindung mit einem Azetylcholinesterasehemmer konnte ein Arzneimittel zur optimierten Behandlung des Morbus Alzheimer und zur Verhütung des Fortschreitens dieser Erkrankung geschaffen werden.

Es herrscht ein Synergismus zwischen Lithiumverbindungen und Azetylcholinesterasehemmern dahingehend vor, daß in dieser fixen Kombination eine synergistische Gesamtwirkung der beiden Einzelwirkstoffe erzielt werden kann. Dies beruht auf der Erkenntnis, daß beide Substanzen, nämlich Lithiumverbindung und Azetylcholinesterasehemmer, in dieselbe Ursachenkette des Morbus Alzheimer eingreifen und somit eine synergistische Wirkung im Sinne einer verstärkten Wirkung per se und einer Zunahme des Ansprechens auf die Behandlung erzielt werden kann. Die gleichgerichteten Effekte von Lithiumverbindungen mit denen von Azetylcholinesterasehemmern beinhalten intrazelluläre Effekte.

Die fixe Kombination einer Lithiumverbindung mit einem Azetylcholinesterasehemmer ermöglicht eine optimierte Behandlung des Morbus Alzheimer. In der Kombination beider Wirkstoffe kann die übliche Dosierung der Einzelkomponenten gesenkt werden, wodurch die Verträglichkeit steigt.

Zweckmäßigerweise übersteigt in den erfindungsgemäßen Arzneimitteln die Dosierung der Komponenten die üblichen Tagesdosen nicht. Das Wirkverhältnis der Lithiumverbindung zu den Azetylcholinesterasehemmern ist so abzustimmen, daß daraus eine Erhöhung der Wirksamkeit bei Morbus Alzheimer resultiert. Das molare Verhältnis zwischen Lithiumverbindungen und Cholinesterasehemmern kann zwischen 100 und 0,01 liegen. Die fixe Kombination wird systemisch, d.h. oral, intravenös oder anders verabreicht. Die fixe Kombination läßt sich in üblicher pharmazeutischer Weise zu den gewünschten Darreichungsformen konfektionieren, wobei Trägerstoffe, Verdünnungsmittel und/oder Hilfsstoffe eingesetzt werden können.

## Patentansprüche

1. Verwendung einer fixen Kombination aus zumindest einer Lithiumverbindung und zumindest einem Azetylcholinesterasehemmer zur Herstellung eines Arzneimittels zur Prophylaxe und Behandlung des Morbus Alzheimer.

2. Verwendung nach Anspruch 1, dadurch gekennzeichnet, daß die zumindest eine Lithiumverbindung ausgewählt ist aus der Gruppe bestehend aus Lithiumazetat, -adipinat, -aspartat, -hydrogenaspartat, -benzoat, -bromid, -chlorid, -glukonat, -karbonat, -orotat, -salizylat, -sulfat,-zitrat.

3. Verwendung nach Anspruch 1, dadurch gekennzeichnet, daß der zumindest eine Azetylcholinesterasehemmer ausgewählt ist aus der Gruppe bestehend aus Physostigmin und Tacrin.

4. Verwendung nach einem der Ansprüche 1 bis 3, dadurch gekennzeichnet, daß die Wirkstoffe in Form systemisch verabreichbarer, insbesondere als oral und/oder intravenös verabreichbare Formulierungen konfektioniert sind.

5. Verwendung nach einem der Ansprüche 1 bis 4, dadurch gekennzeichnet, daß das molare Verhältnis zwischen Lithiumverbindung und Azetylcholinesterasehemmer zwischen 100 und 0,01 liegt.

## Claims

1. Use of a fixed combination of at least one lithium compound and at least one acetylcholinesterase inhibitor for preparing a medicament for the prophylaxis and treatment of Alzheimer's disease.

2. Use according to Claim 1, characterised in that the at least one lithium compound is selected from the group consisting of lithium acetate, lithium adipate, lithium aspartate, lithium hydrogenaspartate, lithium benzoate, lithium bromide, lithium chloride, lithium gluconate, lithium carbonate, lithium orotate, lithium salicylate, lithium sulfate, lithium citrate.

3. Use according to Claim 1, characterised in that the at least one acetylcholinesterase inhibitor is selected from the group consisting of physostigmine and tacrine.

4. Use according to any one of Claims 1 to 3, characterised in that the active substances are processed in the form of systemically administrable formulations, in particular as orally and/or intravenously administrable formulations.

5. Use according to any one of Claims 1 to 4, characterised in that the molar ratio of lithium compound to acetylcholinesterase inhibitor amounts to between 100 and 0.01.

## Revendications

1. Utilisation d'une combinaison fixe d'au moins un composé du lithium et d'au moins un inhibiteur de l'acétylcholinestérase pour préparer un médicament destiné à la prophylaxie et au traitement de la maladie d'Alzheimer.

2. Utilisation selon la revendication 1, caractérisée en ce que le composé du lithium, dont au moins une sorte est présente, est choisi dans le groupe constitué de l'acétate, l'adipinate, l'aspartate, l'hydrogénoaspartate, le benzoate, le bromure, le chlorure, le gluconate, le carbonate, l'orotate, le salicylate, le sulfate, le citrate de lithium.

3. Utilisation selon la revendication 1, caractérisée en ce que l'inhibiteur de l'acétylcholinestérase, dont au moins une sorte est présente, est choisi dans le groupe constitué de la physostigmine et de la tacrine.

4. Utilisation selon l'une des revendications 1 à 3, caractérisée en ce que les principes actifs sont préparés sous la forme de formulations administrables par voie systémique, en particulier administrables par voie orale et/ou intraveineuse.

5. Utilisation selon l'une des revendications 1 à 4, caractérisée en ce que le rapport molaire entre le composé du lithium et l'inhibiteur de l'acétylcholinestérase est situé entre 100 et 0,01.
